# EUROPEAN PATENT APPLICATION

(11) **EP 1 504 716 A1**
(43) Date of publication of application: **09.02.2005**
(21) Application number: 03728067.4
(22) Date of filing: 14.05.2003
(51) Int. Cl.: A61B 5/024, A61B 5/103

(54) **PULSE ABNORMALITY MONITOR AND PULSE ABNORMALITY WARNING SYSTEM**

(30) Priority: 15.05.2002 JP 2002140565
(71) Applicant: MATSUSHITA ELECTRIC INDUSTRIAL CO., LTD., Kadoma-shi, Osaka 571-8501 (JP)
(72) Inventor: TANAKA, Shinji, Ibaraki-shi, Osaka 567-0042 (JP); INOUE, Shigeyuki, Kyotanabe-shi, Kyoto 610-0357 (JP); YAMAMOTO, Hiroshi, Shijyonawate-shi, Osaka 575-0013 (JP)
(74) Representative: Grünecker, Kinkeldey, Stockmair & Schwanhäusser Anwaltssozietät
(86) International application number: PCT/JP2003/005967
(87) International publication number: WO 2003/096892

(57) **Abstract**

A device of automatically judging pulse abnormalities based on a pulse judgment result and a posture judgment result or an activity amount, including means of measuring a pulse wave of a body, means of calculating a pulse of the body based on the pulse wave measured by the measuring means, means of detecting an acceleration of the body, means of detecting and judging a posture or an activity amount of the body based on the acceleration, and means of judging abnormality based on judgment information predetermined according to the calculated pulse information and the detected and judged posture information or the activity amount.

## Description

### Technical Field

The present invention relates to a device and a system that detect the state of a body and issue an alarm at a time of abnormal state.

### Background Art

Care for elderly people has recently received attention against the backdrop of the arrival of an aging society. Particularly special emphasis is placed on care for the elderly having a disease such as a chronic illness and thus it is believed that automatic monitoring on the action of the elderly and automatic reporting made to the outside in the event of an abnormal state are considerably significant in terms of care. Further, in consideration of a recently increasing number of small size households or nuclear families, automatic monitoring for detecting an abnormal state of people as well as the elderly is quite effective in terms of the early detection of an abnormal state. Particularly in the case of people having a heart disease, an abnormal state appears as an abnormal pulse including an increased pulse rate and thus a pulse serves as an index of an abnormal state.

It is necessary to measure a pulse wave to measure a pulse. As a main technique of measuring a pulse wave, a method of measuring a change in blood pressure by wrapping a device around an arm has been widely used in hospitals and at home. Further, methods of measuring a change in light transmission in a blood vessel of an earlobe or a finger have been proposed in recent years. A pulse can be calculated by detecting a change in pulse wave. Besides, a method of judging abnormality based on a judgment result of a pulse has been already known (See Patent Document 1 (Japanese Patent Laid-Open No. 5-189684)).

As described above, a number of methods of measuring a pulse wave and a pulse have been proposed. However, a method of accurately evaluating whether a measured pulse is abnormal or not has not been proposed yet and thus such an evaluation substantially depends upon the decision of an expert such as a doctor under the present circumstances.

Moreover, a pulse to be evaluated is measured in the resting state in a deliberately set measuring time. Thus, it has been impossible to monitor pulse abnormalities all the time in daily life.

### Disclosure of the Invention

An objective of the present invention is to provide a device and a system whereby pulse abnormalities can be accurately detected in daily life.

It is said that the range of normal pulses is varied for each posture of a person. According to human engineering, it is said that normal pulses in an upright position range from 90 to 100 pulses/minute, normal pulses in a seated position range from 60 to 100 pulses/minute, and normal pulses in a lie position range from 40 to 60 pulses/minute. Thus, the posture of a body and a change in activity amount are calculated along with a pulse and it is judged whether or not the pulse is within the normal range in the posture and the activity amount, so that pulse abnormalities can be detected with accuracy. Further, detected information about pulse abnormalities is automatically transmitted to the doctor and family of a patient, so that the pulse abnormalities of the patient can be evaluated remotely. Moreover, mobile phones are set as transmitting terminals, so that the state can be. automatically monitored by the doctor and family even during travel.

A first invention is an abnormal pulse monitor, characterized by comprising:
pulse wave measuring means of measuring a pulse wave of a body,
pulse calculating means of calculating a pulse of the body based on a waveform of the pulse wave measured by the pulse wave measuring means;
acceleration detecting means of detecting an acceleration of the body,
posture deciding means which detects and decide a posture of the body based on the acceleration detected by the acceleration detecting means, and
abnormality judging means of judging abnormality based on predetermined judgment information from pulse information calculated by the pulse calculating means. and posture information decided by the posture deciding means.

A second invention is an abnormal pulse monitor, characterized by comprising:
pulse wave measuring means of measuring a pulse wave of a body,
pulse calculating means of calculating a pulse of the body based on a waveform of the pulse wave measured by the pulse wave measuring means,
acceleration detecting means of detecting an acceleration of the body,
activity amount judging means of judging an activity amount of the body based on the acceleration detected by the acceleration detecting means, and
abnormality judging means of judging abnormality, based on predetermined judgment information, frompulse information calculated by the pulse calculating means and the activity amount decided by the posture judging means.

A third invention is an abnormal pulse monitor characterized by comprising:
pulse wave measuring means of measuring a pulse wave of a body,
pulse calculating means of calculating a pulse of the body based on a waveform of the pulse wave measured by the pulse wave measuring means,
acceleration detecting means of detecting an acceleration of the body,
posture deciding means of deciding a posture of the body based on the acceleration detected by the acceleration detecting means,
activity amount judging means of judging an activity amount based on the acceleration, and
abnormality judging means of judging abnormality based on predetermined judgment information from a combination of the calculated pulse information, the detected and judged posture information, and the activity amount.

A fourth invention is the abnormal pulse monitor according to any one of inventions 1 to 3, characterized by further comprising notifying means of notifying a device user of a judgment result from the abnormality judging means.

A fifth invention is the abnormal pulse monitor according to any one of inventions 1, and 3 to 4, characterized in that the abnormality judging means judges abnormality when the pulse calculated by the pulse calculating means is larger than a first pulse threshold value predetermined according to the posture information judged by the posture judging means.

A sixth invention is the abnormal pulse monitor according to any one of inventions 1, and 3 to 5, characterized in that the abnormality judging means judges abnormality when the pulse calculated by the pulse calculating means is smaller than a second pulse threshold value predetermined according to the posture information obtained by the posture judging means.

A seventh invention is the abnormal pulse monitor according to any one of inventions 2 to 4, characterized in that the abnormality judging means judges abnormality when the pulse calculated by the pulse calculating means is larger than a third pulse threshold value predetermined according to the activity amount obtained by the activity amount judging means.

An eighth invention is the abnormal pulse monitor according to any one of inventions 2 to 4 and 7, characterized in that the abnormality judging means judges abnormality when the pulse calculated by the pulse calculating means is smaller than a fourth pulse threshold value predetermined according to the activity amount obtained by the activity amount judging means.

A ninth invention is the abnormal pulse monitor according to any one of inventions 3 and 4, characterized in that the abnormality judging means judges abnormality when the pulse calculated by the pulse calculating means is larger than a fifth pulse threshold value predetermined according to a combination of the posture information obtained by the posture judging means and the activity amount obtained by the activity amount judging means.

A tenth invention is the abnormal pulse monitor according to any one of inventions 3 and 4, characterized in that the abnormality judging means judges abnormality when the pulse calculated by the pulse calculating means is smaller than a sixth pulse threshold value predetermined according to a combination of the posture information obtained by the posture judging means and the activity amount obtained by the activity amount judging means.

An eleventh invention is the abnormal pulse monitor according to any one of inventions 2 to 4 and 7 to 10, characterized in that the monitor further comprises information storage means of temporarily storing the pulse information, the posture information, and the activity information, and the abnormality judging means judges the pulse to be abnormal based on a time series variation in at least a part of the information stored in the information storage means.

A twelfth invention is the abnormal pulse monitor according to any one of inventions 3 to 4, 9, and 10, characterized in that the monitor further comprises information storage means of temporarily storing the judgment result, and the abnormality judging means judges the pulse to be abnormal based on a time series variation of the judgment result stored in the information storage means.

A thirteenth invention is the abnormal pulse monitor according to any one of inventions 1, 3 to 6, 9 and 10, characterized in that the monitor further comprises information storage means of temporarily storing the judgment result, and the abnormality judging means judges the pulse to be abnormal based on a frequency in a specified time of the judgment result stored in the information storage means.

A fourteenth invention is the abnormal pulse monitor according to any one of inventions 1 to 13, characterized by further comprising abnormality transmitting means of transmitting abnormality judgment information when the abnormality judging means judges abnormality.

A fifteenth invention is an abnormal pulse alarming system, characterized by comprising the abnormal pulse monitor of invention 14, and
abnormality receiving means of communicating with the abnormality receiving means and receiving the abnormality judgment information.

A sixteenth invention is the abnormal pulse alarming system according to invention 15, characterized by further comprising the information indicating means which is connected to the abnormality receiving means and indicates the abnormality judgment information.

A seventeenth invention is the abnormal pulse alarming system according to invention 15 or 16, characterized by further comprising alarming means which is connected to the abnormality receiving means and issues an alarm to a user when the abnormality receiving means receives the abnormality judgment information.

An eighteenth invention is the abnormal pulse alarming system according to any one of inventions 15 to 17, characterized in that the abnormality transmitting means and the abnormality receiving means further comprise encrypting means of encrypting the abnormality judgment information.

A ninteenth invention is the abnormal pulse alarming system according to any one of inventions 15, 16 and 18, characterized in that the abnormality receiving means further comprises information indicating/selecting means of indicating information on the information indicating means which is specified beforehand based on the abnormal pulse monitor having transmitted the abnormality judgment information.

A twentieth invention is the abnormal pulse alarming system according to any one of inventions 15, 17, and 18, characterized in that the abnormality receiving means further comprises alarm issuing/selecting means of issuing an alarm to the alarming means which is specified beforehand based on the abnormal pulse monitor having transmitted the abnormality judgment information.

A twenty-first invention is the abnormal pulse alarming system according to any one of inventions 15, 16, 18 and 19, characterized in that the system further comprises abnormality information accumulating means which connects the abnormality receiving means and the information indicating means and accumulates the abnormality information, and a user makes an access to the abnormality information accumulating means at any given time to obtain abnormality information, so that the information indicating means indicates the abnormality information.

A twenty-second invention is the abnormal pulse alarming system according to any one of inventions 15 to 21, characterized in that the abnormality judgment information includes at lease one of the pulse information, the posture information, and the activity amount information.

A twenty-third invention is the abnormal pulse monitor according to any one of inventions 1 to 3, characterized in that when the posture judging means or the activity amount judging means finds a change in posture or activity amount, the abnormality judging section stops judgment for a certain time period from the change.

A twenty-fourth invention is a program of causing a computer to function as the pulse calculating means of calculating a pulse of a body based on a waveform of a pulse wave measured by the pulse wave measuring means, the posture judging means of judging a posture of the body based on an acceleration detected by the acceleration detecting means, the activity amount judging means of judging an activity amount based on the acceleration, and abnormality judging means of judging abnormality based on judgment information predetermined according to a combination of the calculated pulse information, the detected and judged posture information, and the activity amount, of the abnormal pulse monitor accoring to invention 3.

A twenty-fifth invention is a recording medium carrying the program of invention 24, the recording medium being processed by a computer.

According to the present invention, it is possible to automatically detect pulse abnormalities while a person requesting a pulse evaluation lives an ordinary life. Further, pulse abnormalities obtained from the device are automatically transmitted to the doctor of a patient or the like, so that the pulse abnormalities of the patient can be remotely evaluated with high effectiveness.

### Brief Description of the Drawings

Figure 1 is a schematic structural diagram showing an abnormal pulse monitor according to Embodiment 1 of the present invention;
Figure 2 shows a person wearing the abnormal pulse monitor and an example of measurement results of a pulse wave according to Embodiment 1 of the present invention;
Figure 3 shows a judgment example of a pulse warning in abnormal pulse judging means according to Embodiment 1 of the present invention;
Figure 4 shows a judgment example of a pulse warning in the abnormal pulse judging means according to Embodiment 1 of the present invention;
Figure 5 shows a judgment example of a pulse warning in the abnormal pulse judging means according to Embodiment 1 of the present invention;
Figure 6 shows flows of judging an abnormal pulse according to Embodiment 1 of the present invention;
Figure 7 shows a flow of judging an abnormal pulse according to Embodiment 1 of the present invention;
Figure 8 shows a flow of judging an abnormal pulse according to Embodiment 1 of the present invention;
Figure 9 shows a flow of judging an abnormal pulse according to Embodiment 1 of the present invention;
Figure 10 is a conceptual diagram showing judgment on an abnormal pulse according to Embodiment 1 of the present invention;
Figure 11 shows the orientations of an acceleration sensor when a posture is changed according to Embodiment 1 of the present invention;
Figure 12 shows the output of the acceleration sensor when the posture is changed according to Embodiment 1 of the present invention;
Figure 13 shows the output of the acceleration sensor when the posture is changed according to Embodiment 1 of the present invention;
Figure 14 is a schematic structural diagram showing an abnormal pulse alarming system according to Embodiment 2 of the present invention;
Figure 15 is a schematic structural diagram showing an abnormal pulse alarming system according to Embodiment 3 of the present invention; and
Figure 16 is a schematic structural diagram showing an abnormal pulse alarming system according to Embodiment 4 of the present invention.

### [Description of Symbols]

- 11: Acceleration sensor
- 12: Posture judging section
- 13: Rheometer
- 14: Pulse calculating section
- 15: Memory
- 16: Abnormal pulse judging section
- 17: Abnormal pulse monitor
- 21: User
- 31: Pulse information
- 32: Posture information
- 91: User
- 92: Upright position
- 93: Seated position
- 94: Lie position
- 121: Abnormality transmitting section
- 122: Abnormality receiving means
- 123: Alarm receiver
- 131: PHS
- 132: Device user
- 133: Public line network
- 134: Public line base station
- 141: Internet browser
- 142: Service provider
- 143: Server

### Best Mode for Carrying Out the Invention

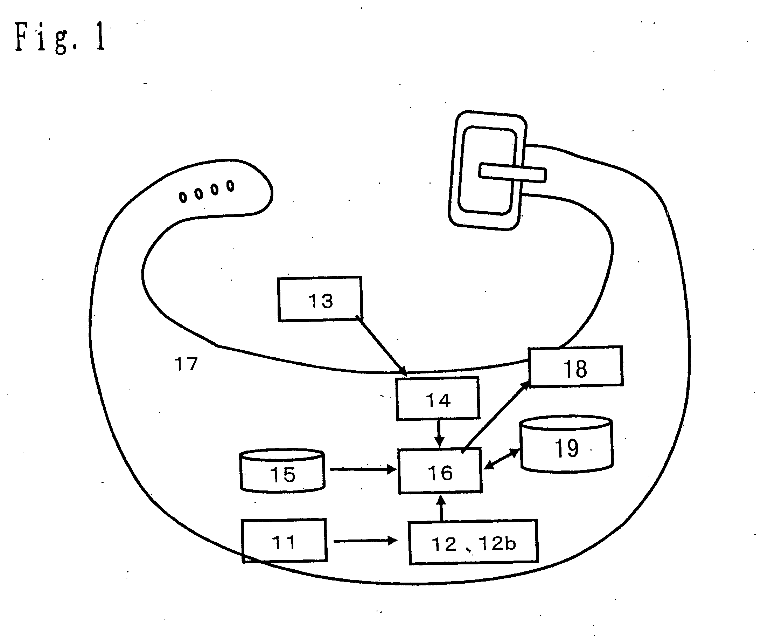

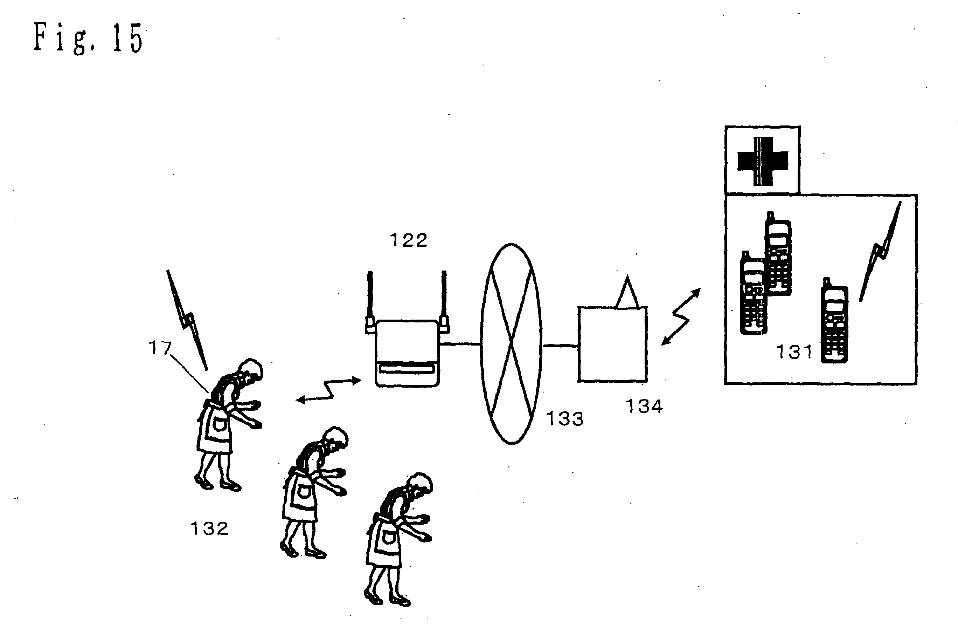

Referring to Figures 1 to 15, the following will describe embodiments of the present invention.

### (Embodiment 1)

An abnormal pulse monitor of Embodiment 1 will be discussed below in accordance with the accompanying drawings.

Figure 1 is a schematic structural diagram showing the abnormal pulse monitor according to Embodiment 1 of the present invention. The following will describe the configuration of the present embodiment. Embodiment 1 is constituted of an acceleration sensor 11 of detecting a gravitational acceleration, a posture judging section 12 of judging the posture of a body based on a gravitational acceleration obtained by the acceleration sensor 11, a rheometer 13 of measuring a pulse wave, a pulse calculating section 14 of calculating the pulse of a body based on a change in blood flow that is obtained by the rheometer, a memory 15 of storing judgment information for judging an abnormal pulse, and an abnormal pulse judging section 16 of judging the abnormality of a pulse by referring to the memory 15 based on pulse information calculated by the pulse calculating section 14 and posture information judged by the posture judging section 12.

An abnormal pulse monitor 17 is attached to a body. It is desirable to select a waist or the like on a trunk as an attaching position. Notifying means 18 maybe further provided which notifies a monitor user of abnormality when a pulse is judged to be abnormal. Attention may be called by using sound and light.

Further, an activity amount judging section 12b may be provided instead of the posture judging section 12 to judge the activity amount of a body based on an acceleration obtained by the acceleration sensor 11. Alternatively, both of the posture judging section 12 and the activity amount judging section 12b may be provided. When both of the sections are provided, the posture and the activity amount can be judged at the same time by a single acceleration sensor 11.

Information storage means 19 connected to the abnormal pulse judging section 16 may be further provided to temporarily store pulse information and/or posture information and/or an activity amount.

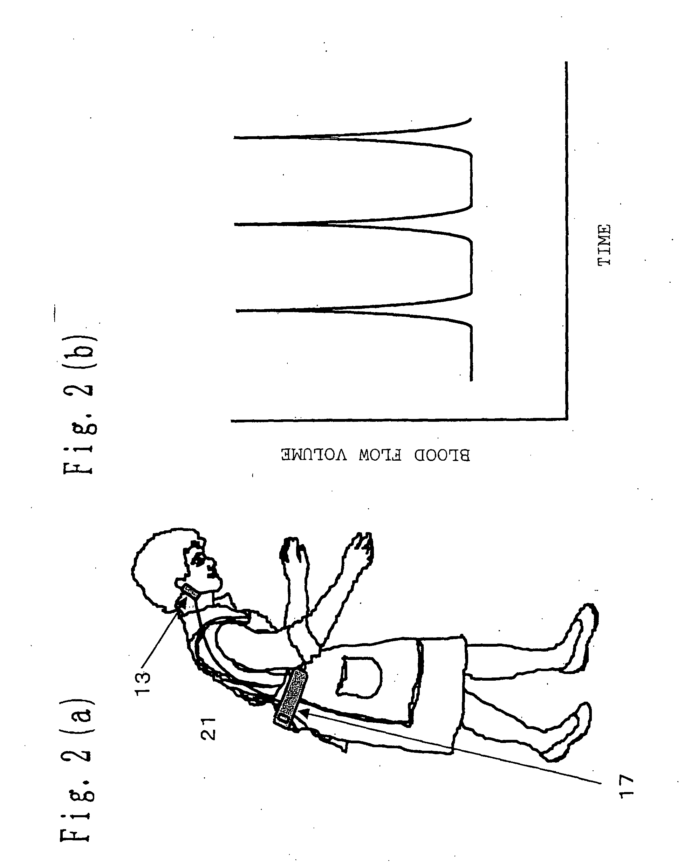

Figure 2(a) shows a person wearing the abnormal pulse monitor around his/her waist. A user 21 wears the abnormal pulse monitor 17 around his/her waist and separately wears the rheometer 13. The rheometer 13 is, for example, a rheometer using a method of measuring a blood flow volume based on a light transmittance in a blood vessel of an earlobe. Figure 2 shows such a kind of rheometer. The pulse calculating section 14 in the abnormal pulse monitor 17 calculates a pulse based on a pulse wave obtained by the rheometer 13. When the judgment result of the abnormal pulse judging section 16 indicates that a pulse becomes abnormal, the result is informed to the user or an alarm is issued by the abnormal pulse monitor 17 to the outside. In response to the alarm, the user 21 makes contact with the doctor, thereby preventing amore dangerous situation.

Besides, Figure 2(b) shows an example of a waveform regarding the measurement results of a blood flow volume in the rheometer. According to the measurement results of Figure 2(b), the pulse calculating section 14 in the abnormal pulse monitor 17 calculates a pulse by detecting, for example, a peak number in a unit time.

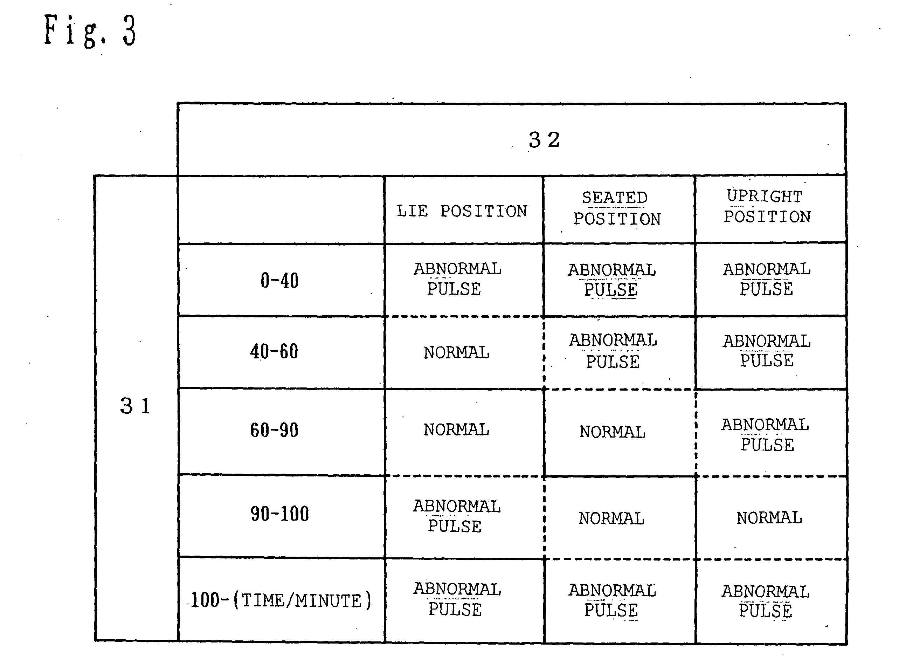

Figure 3 shows judgment examples of abnormal pulses in the abnormal pulse judging section 16. Abnormal pulses are judged based on the combination of pulse information 31 in the pulse calculating section 14 and posture information 32 in the posture judging section 12. According to human engineering, normal pulses in an upright position range from 90 to 100 pulses/minute, normal pulses in a seated position range from 60 to 100 pulses/minute, and normal pulses in a lie position range from 40 to 60 pulses/minute.

An example of a judgment criterion on abnormality is shown in which abnormal pulses are judged when the posture information 32 indicates an upright position and the pulse information 31 indicates 0 to 90 pulses/minute or 100 pulses/minute or more, when the posture information 32 indicates a seated position and the pulse information 31 indicates 0 to 60 pulses/minute or 100 pulses/minute or more, and when the posture information 32 indicates a lie position and the pulse information 31 indicates 0 to 40 pulses/minute or 90 pulses/minute or more. In this example, both of the upper limit and the lower limit are indicated as normal ranges of pulses for each of the postures. Only one of the upper limit and the lower limit may be used to judge abnormality. Further, differences among individuals may be considered beforehand to change the range of normal pulses. Alternatively, the range of normal pulses may be automatically changed by sequential learning.

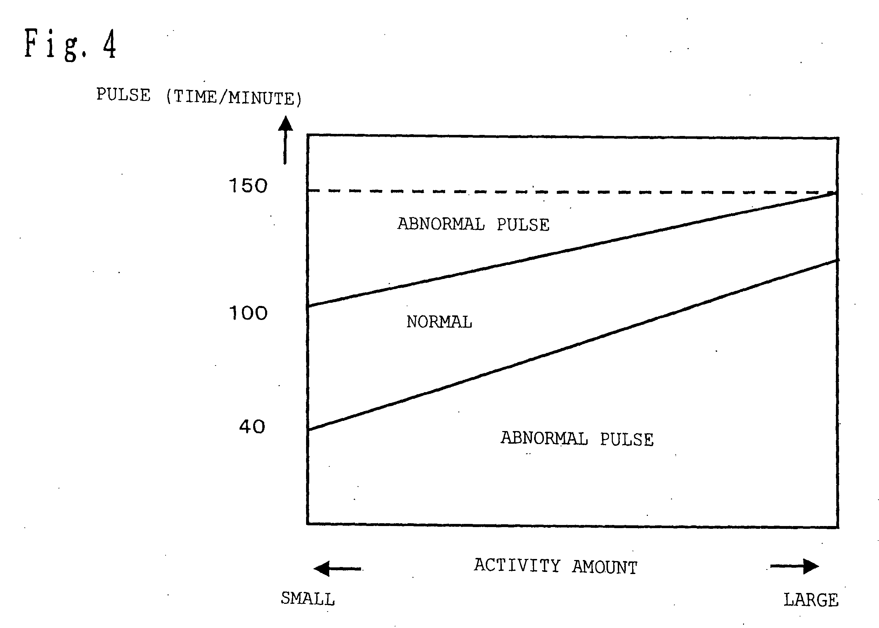

Figure 4 shows a judgment example of abnormal pulses in the abnormal pulse judging section. Abnormal pulses are judged based on the combination of the pulse information 31 in the pulse calculating section 14 and an activity amount 41 in the activity amount judging section 18. For example, the activity amount 41 can be set by categorizing, based on a predetermined specified value, the adding results of changes in acceleration during a certain period of time.

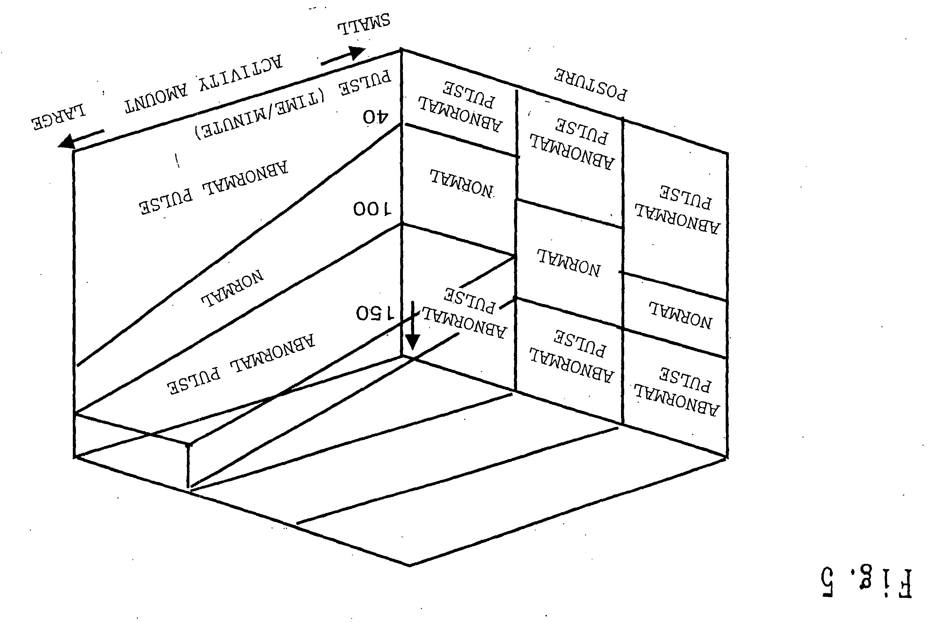

Figure 5 shows a judgment example of abnormal pulses in the abnormal pulse judging section. Abnormal pulses are judged based on the combination of the pulse information 31 in the pulse calculating section 14 and the posture information 32 and the activity amount 41 in the posture judging section 12 and the activity amount judging section 18. For example, when postures are the same but actions are different at some point in time, the states of pulses should be different. When a comparison is made between body recovery in an upright position and body recovery in a lie position after an action is performed for a certain time, it is readily understood that the lie position makes a larger recovery. Thus, it is understood that in the case of an action continued for a certain period of time, a possible pulse frequency is changed according to the posture as well as an activity amount, thereby detecting an abnormal pulse according to the posture after an action is performed. Further, since a pulse increase corresponding to an activity amount and a posture may include differences among individuals, the range of normal pulses may be changed for each person by considering differences among individuals beforehand. Alternatively, the range of normal pulses may be automatically changed by sequential learning.

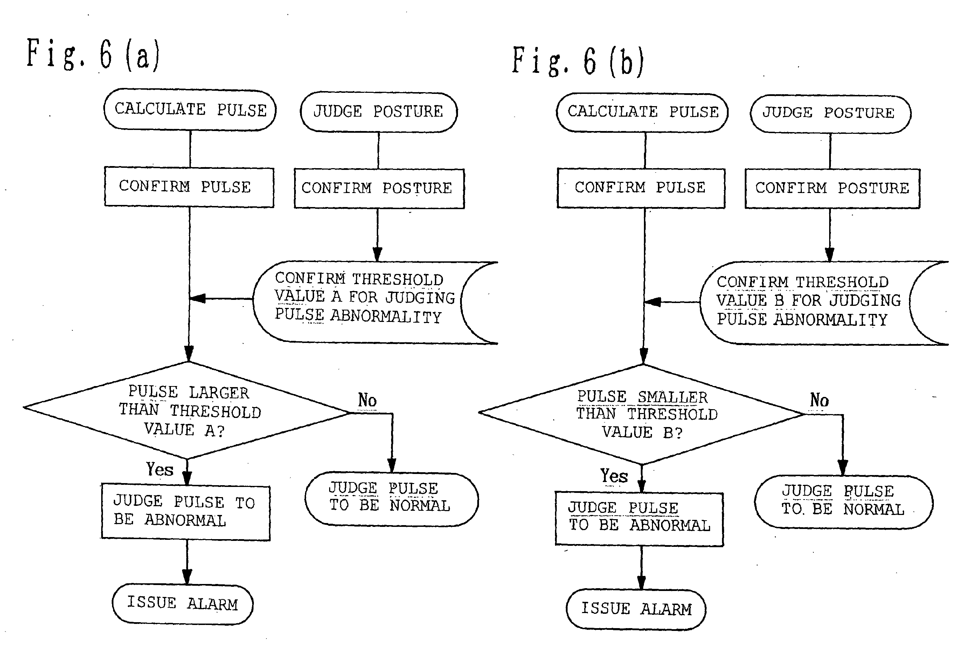

Figures 6(a) and 6(b) show flows of judging an abnormal pulse in the abnormal pulse judging section 16. First, the posture information 32 judged in the posture judging section 12 and the pulse information 31 calculated in the pulse calculating section 14 are obtained. According to the obtained posture information 32, a predetermined threshold value A or threshold value B is extracted as a judgment threshold value from the memory 15, and the threshold value A or threshold value B is compared with the pulse information 31 of the pulse calculating section 14. When the pulse information 31 is larger than the threshold value A or smaller than the threshold value B as a result of the comparison, the pulse is judged to be abnormal. Thus, for example, the result is left for the user or alarming means is started to issue an alarm.

Although the posture information 32 is used in these flows, the activity amount 41 may be used with additionally specified threshold values A' and B'.

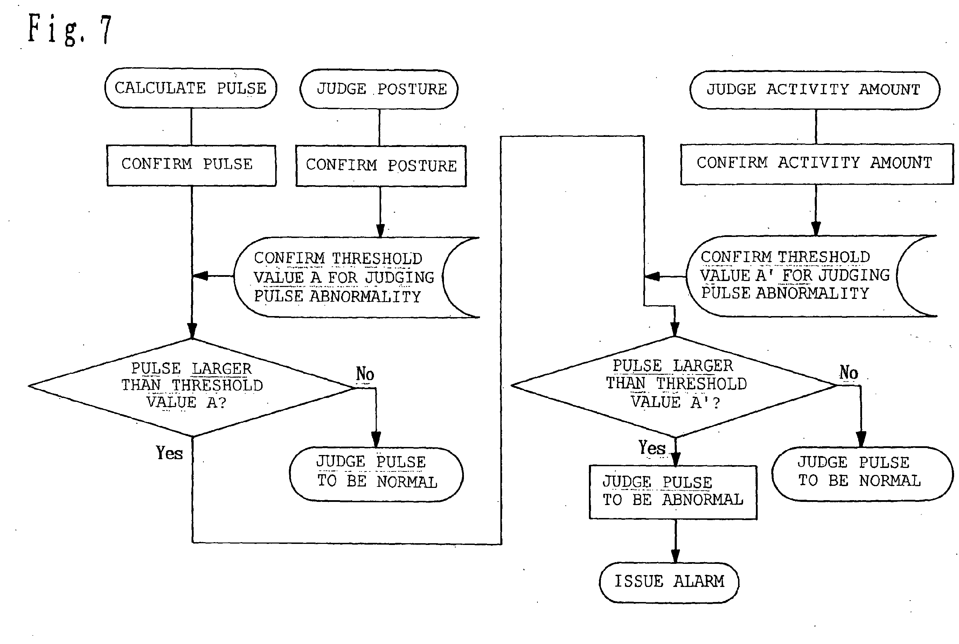

Figure 7 shows a flow of judging an abnormal pulse in the abnormal pulses judging section 16. First, the posture information 32 judged in the posture judging section 12, the activity amount 41 judged in the activity amount judging section 18, and the pulse information 31 calculated in the pulse calculating section 14 are obtained.

According to the obtained posture information 31, a predetermined threshold value A or threshold value B is extracted as a judgment threshold value from the memory 15, and the threshold value A or threshold value B is compared with the pulse information 31 of the pulse calculating section 14.

When the pulse information 31 is larger than the threshold value A or smaller than the threshold value B as a result of the comparison, a predetermined threshold value A' or threshold value B' is extracted as a judgment threshold value from the memory according to the obtained activity amount 41, and the threshold value A' or threshold value B' is compared with the pulse information 31 of the pulse calculating section 14. When a pulse is found to be abnormal as a result of the comparison, abnormality is judged. Further, the alarming means may be started to issue an alarm.

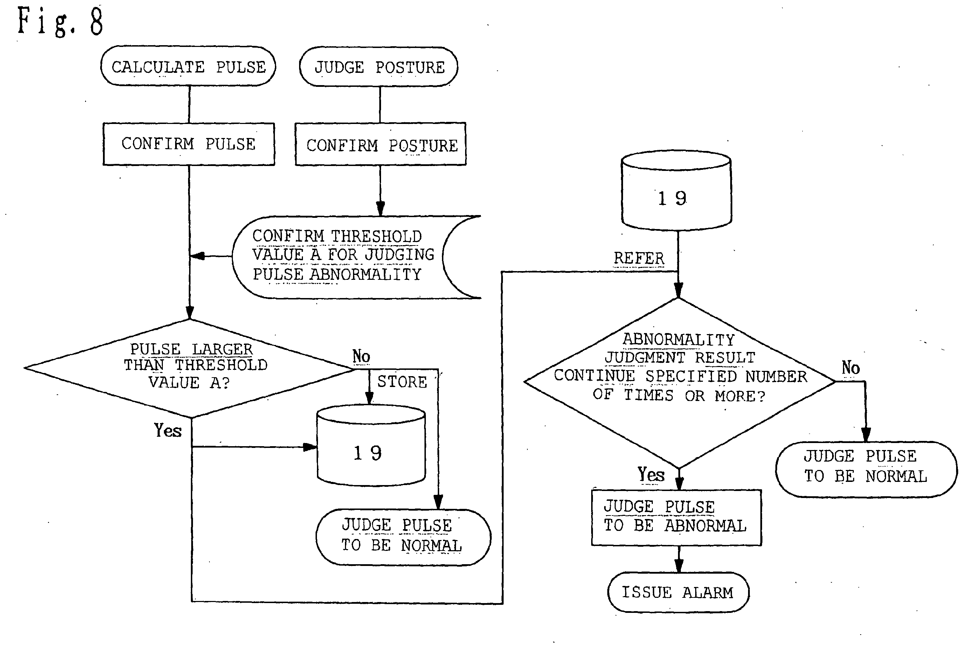

Figure 8 shows a flow of judging an abnormal pulse in the abnormal pulse judging section 16. First, the posture information 32 judged in the posture judging section 12 and the pulse information 31 calculated in the pulse calculating section 14 are obtained. According to the obtained posture information 32, a predetermined threshold value A or threshold value B is extracted as a judgment threshold value from the memory 15, and the threshold value A or threshold value B is comparedwith the pulse information 31 of the pulse calculating section 14. When the pulse information 31 is larger than the threshold value A or smaller than the threshold value B as a result of the comparison, the pulse is judged to be abnormal and the result is stored in the information storage means 19 for a certain period of time.

Such a judgment is repeated every 1/60 second.

When abnormality is judged, for example, three times in succession, pulse abnormality is judged and an alarm is issued.

In this way, when abnormality is judged, it is possible to prevent an erroneous judgment caused by noise.

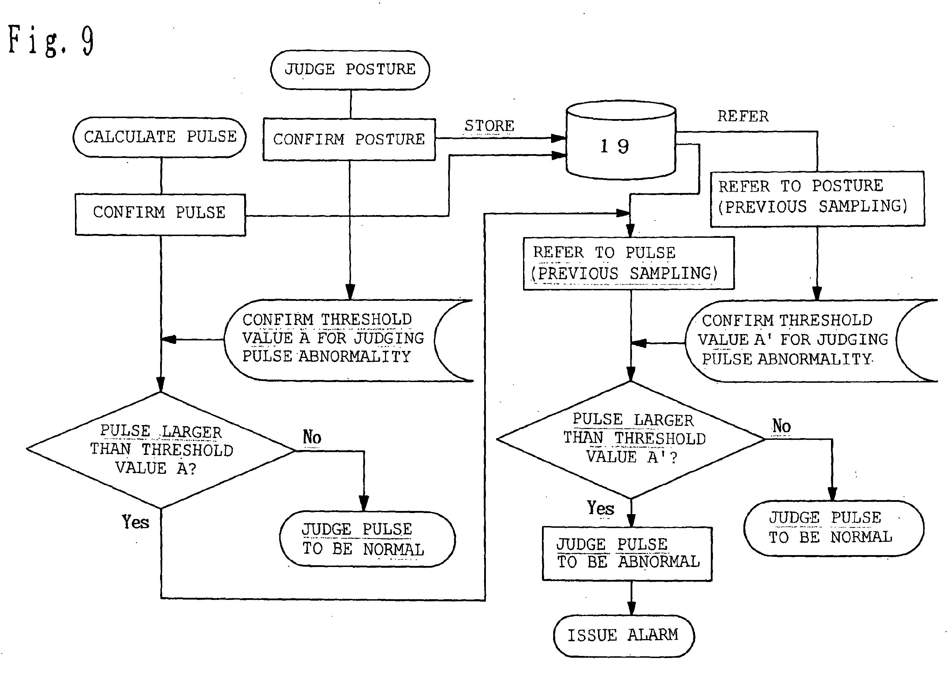

Figure 9 is a flow of judging an abnormal pulse in the abnormal pulse judging section 16.

First, the posture information 32 judged in the posture judging section 12, the activity amount 41 judged in the activity amount judging section 18, and the pulse information 31 calculated in the pulse calculating section 14 are obtained. The obtained posture information 32, activity amount 41, and pulse information 31 are stored in the information storage means 19 for a certain period of time.

According to the obtained posture information 32, a predetermined threshold value A or threshold value B is firstly extracted as a judgment threshold value from the memory 15, and the threshold value A or threshold value B is compared with the pulse information 31 of the pulse calculating section 14.

When the pulse information 31 is larger than the threshold value A or smaller than the threshold value B as a result of the comparison, a predetermined threshold value A' or threshold value B' is then extracted as a judgment threshold value from the memory 15 according to the obtained activity amount 41, and the threshold value A' or threshold value B' is compared with the pulse information 31 of the pulse calculating section 14. When a pulse is found to be abnormal as a result of the comparison, temporary abnormality is judged as an abnormality judgment result of the current period.

When temporary abnormality is judged, among the posture information 32, activity amount 41, and pulse information 31 that are stored in the information storage means 19, reference is made to information obtained in the previous period serving as a sampling period, and the information is used to perform the same judgment. As a result, if the judgment result of the previous period is also temporary abnormality, a state at that time is confirmed to be an abnormal state. With this operation, it is possible to prevent erroneous detection caused by a momentary abnormality judgment, achieving an abnormality judgment with high accuracy. In this case, abnormality is confirmed by making a judgment again in the previous period. As a matter of course, information obtained two periods before or three periods before may be stored in the information storage means 19 to make a judgment again. Further, regardless of whether or not the judgment result of the current period is abnormal, abnormality judgment results may be sequentially stored in the information storage means 19. When temporary abnormality is judged in the subsequent period, reference may be made only to the abnormality judgment results. In this case, although the information storage means 19 requires a large storage capacity, a processing load can be reduced. In this way, it is believed that when an action is continuously performed for a certain period of time in daily life, possible pulse frequencies are changed according to the activity amount 41, so that abnormal pulses can be detected according to an activity state. Moreover, since it is believed that a pulse increase corresponding to the activity amount includes differences among individuals, the differences among individuals may be considered beforehand and a threshold value may be changed for each person. Alternatively, the threshold value may be automatically changed by sequential learning.

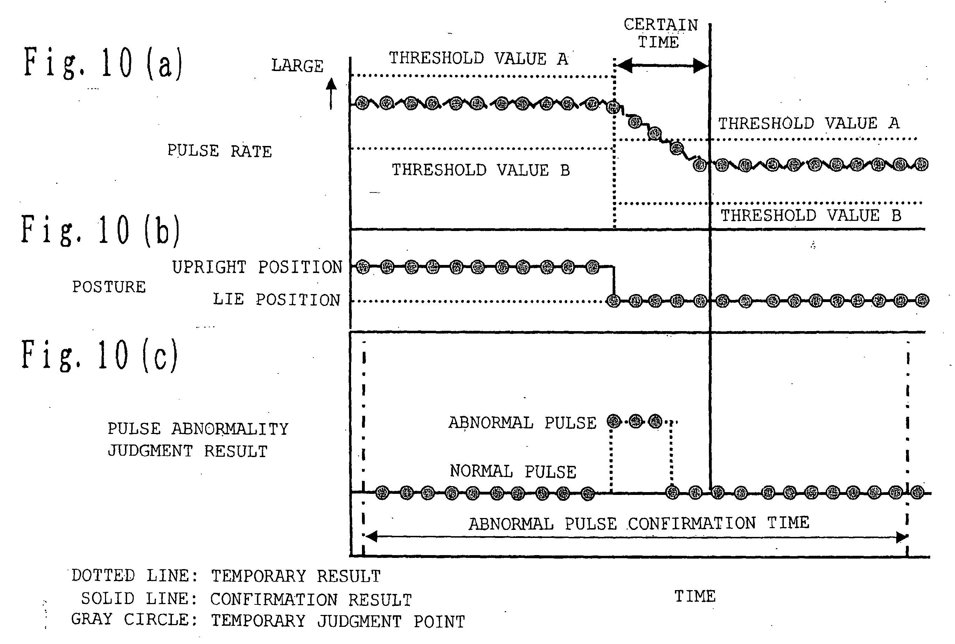

Figure 10 is a conceptual diagram showing judgment on abnormal pulses in the abnormal pulse judging section 16. First, pulse abnormality is judged based on the combination of the pulse information 31 in the pulse calculating section 14 and the posture information 32 in the posture judging section 12. Pulse abnormality judgment results are sequentially stored in the information storage means 19.

Figure 10(a) shows pulse abnormality results stored in the information storage means 19. A horizontal axis indicates a time base and a vertical axis indicates abnormality judgment results. Namely, the judgment results are shown in time sequence.

Moreover, Figure 10(b) shows an actual change in action during that period. A lie position after a vigorous action is indicated as an example. According to an actual change in pulse, it takes a long time to reduce a pulse rate after the action is stopped and thus a pulse change and a posture change are obtained as shown in Figure 10. At this moment, since the pulse rate is not fully reduced immediately after the vigorous action, the abnormal pulse judging section 16 judges pulse abnormality. Thus, the abnormality judgment result is stored for a certain time period and the frequency of judging abnormal pulses is calculated regarding the judgment results during that time, so that momentary results of abnormality judgment can be eliminated and thus pulse abnormality can be judged with high accuracy.

Besides, when the posture is changed, the change can be judged by the posture judging section 12. In this case, the abnormal pulse judging section 16 may stop making a judgment for a certain time period after the change in order to prevent an erroneous judgment.

Further, not only when the posture is changed but also when the activity amount is changed, it is desirable to similarly stop making an abnormality judgment for a certain period of time.

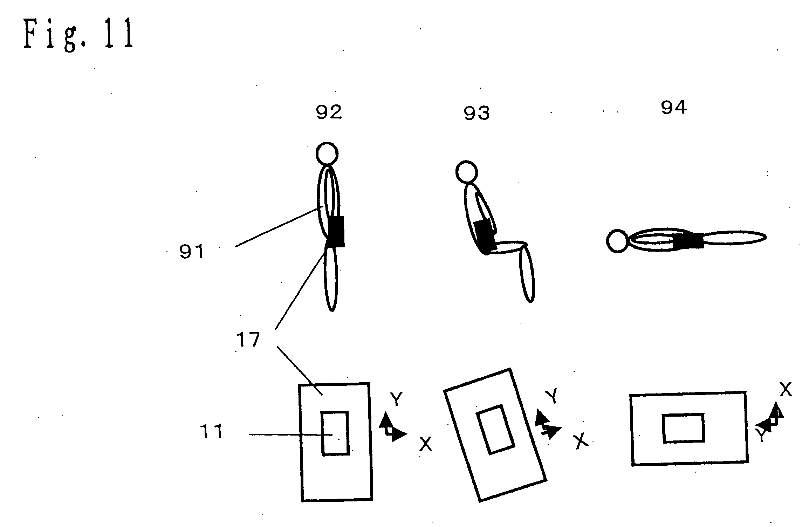

Figure 11 is a schematic view showing that a change in posture is detected by using the acceleration sensor 11. Additionally, the acceleration sensor 11 is fixed on the abnormal pulse monitor 17, has sensitivity in at least two directions of a forward direction and a gravity direction, and is capable of detecting a gravitational acceleration. It is assumed that the abnormal pulse monitor 17 having the acceleration sensor 11 is attached to the waist of a user 91. The posture judging section 12 in the abnormal pulse monitor 17 processes an output which is proportionate to the magnitude of a gravitational acceleration changing according to the inclination of the acceleration sensor 11. Figure 10 shows an upright position 92, a seated position 93, and a lie position (face up) 94 from the left. It is assumed that in the upright position 92, the front of a body is directed along the x axis and the gravity direction of the body is directed along the y axis. The posture of the body is judged by detecting gravitational acceleration components from the outputs of the x axis and the y axis.

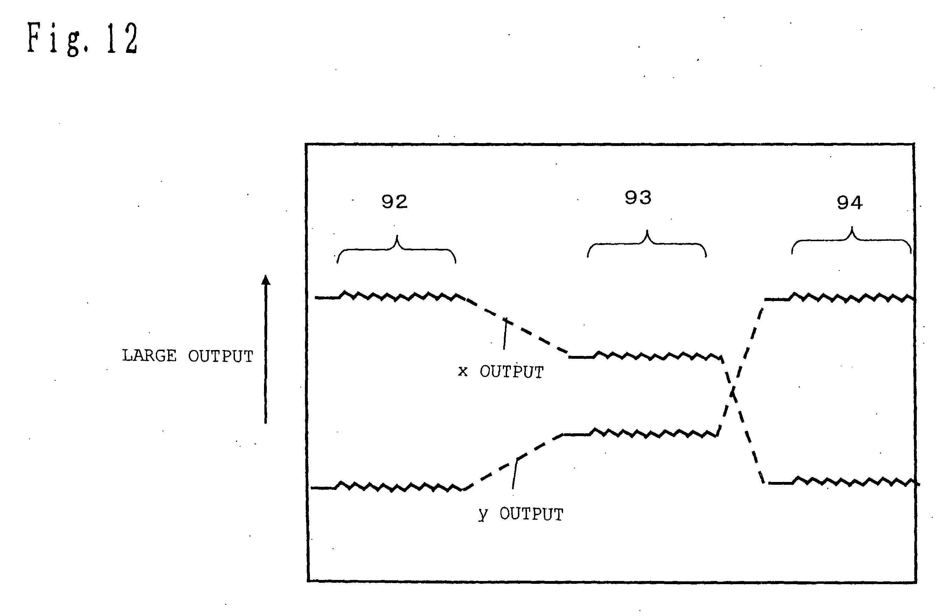

Figure 12 shows the output of the acceleration sensor when a change in posture is detected using the acceleration sensor. The used acceleration sensor is capable of detecting a gravitational acceleration. Figure 11 shows the outputs of the acceleration sensor at the upright position 92, the seated position 93, and the lie position 94 from the left. The upper direction of the graph indicates a larger output from the acceleration sensor. It is assumed that the output is increased as a larger gravitational acceleration is applied to the acceleration sensor. The posture of the body is judged by detecting gravitational acceleration components from the outputs of the x axis and the y axis.

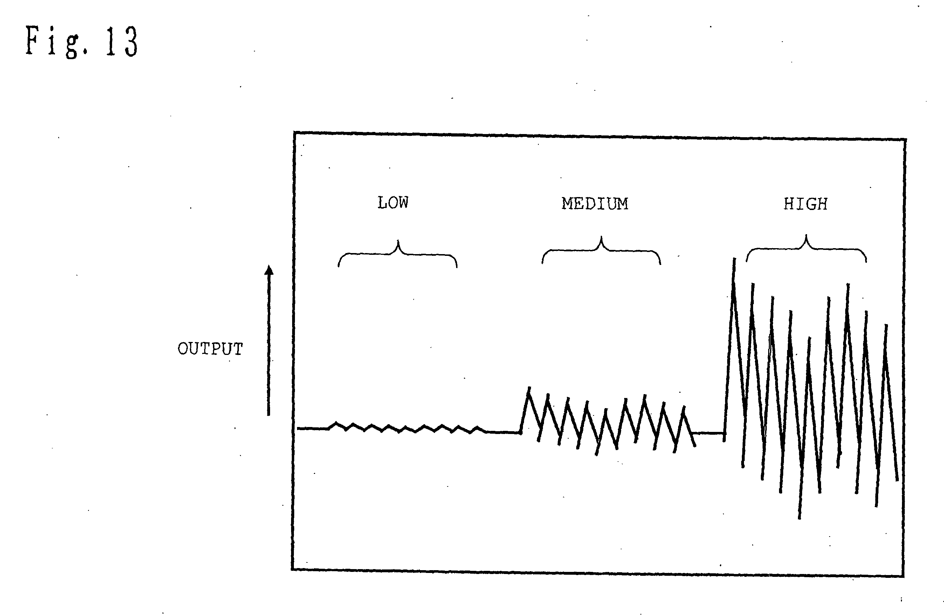

Figure 13 shows the output of the acceleration sensor when an activity amount is detected using the acceleration sensor. When only an activity amount is detected, it is not always necessary to use an acceleration sensor capable of detecting a gravitational acceleration component and thus an acceleration sensor capable of detecting an acceleration change component is enough. Figure 13 shows the output of the acceleration sensor when an activity amount is increased from the left. The activity amount can be calculated in the activity amount judging section by adding primary differential results of the output from the acceleration sensor for a certain period of time and classifying the results. The upper direction of the graph indicates a larger output from the acceleration sensor. The activity amount of the body is judged by detecting gravitational acceleration components from the outputs of the x axis and the y axis.

### (Embodiment 2)

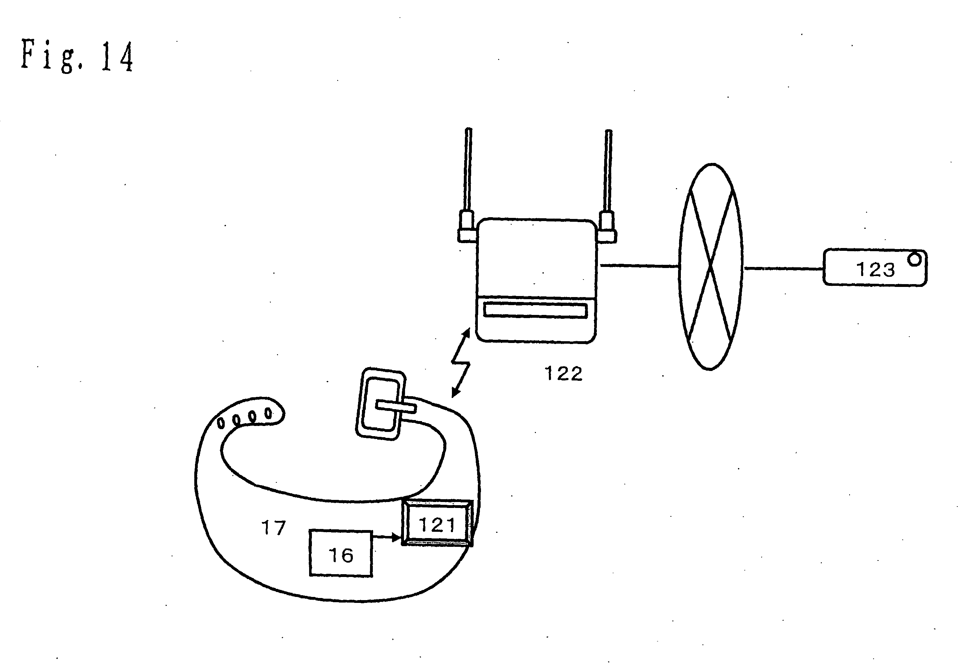

An abnormal pulse monitor of Embodiment 2 will be discussed below in accordance with the accompanying drawings. Figure 14 is a schematic structural diagram showing an abnormal pulse alarming system according to Embodiment 2 of the present invention.

The configuration of Embodiment 2 will be discussed below. Embodiment 2 is constituted of an abnormal pulse monitor 17 configured as Figure 1, an abnormality transmitting section 121 which is connected to an abnormal pulse judging section 16 in the abnormal pulse monitor 17 and carries out radio communication when a pulse is judged to be abnormal by the abnormal pulse judging section 16, abnormality receiving means 122 of receiving a signal indicating abnormality transmitted from the abnormality transmitting section, and an alarm receiver 123 which is operated when the abnormality receiving means 122 receives an abnormal signal.

The abnormality receiving means 122 and the abnormal pulse monitor 17 communicate with each other via radio waves. In Figure 14, a device indicated as the alarm receiver 123 can be set as, for example, a dedicated terminal installed in an outside hospital and so on. With this configuration, it is possible to automatically make a notification to the hospital and so on and thus the user does not have to make a notification. Moreover, the following system is also applicable: the abnormal pulse monitor 17 further comprises a speaker to inquire after the user from the abnormality receiving means 122 through the speaker when the abnormality receiving means 122 receives a judgment result of an abnormal pulse. Alternatively, the abnormal pulse monitor 17 may further comprise a switch to add a function of permitting the user to make a notification of his/her own will. In Figure 14, the alarm receiver 123 is indicated as a device connected to the abnormal pulse monitor 17. An operation may be performed after replacing the device with information indicating means.

Besides, the information includes some pieces of personal information. Thus, when a system using a public communication network is constructed in consideration of privacy in communication with the alarm receiver., an inexpensive system effective for information security can be obtained by adding a function of encrypting information in the abnormality receiving means 122 beforehand. As a communication form between the abnormality receiving means 122 and the abnormal pulse monitor 17, radio communication is desirable in view of restriction imposed when the device is mounted. However, wire communication is also applicable. Regarding the radio communication format, while the system can be constructed so as to be separated from other radio communication networks by using a dedicated specification of radio communication, the system may be constructed so as to have affinity with other networks by using general radio transmission of Bluetooth, a wireless LAN, and so on and using a telephone line such as a PHS network.

### (Embodiment 3)

An abnormal pulse monitor of Embodiment 3 will be discussed in accordance with the accompanying drawings. Figure 14 is a schematic structural diagram showing an abnormal pulse alarming system according to Embodiment 3 of the present invention. The configuration of Embodiment 3 will be discussed below.

According to Embodiment 3, a plurality of PHSs 131 are set as the alarm receivers of the system shown in Figure 14. Unique ID information is added to the devices of users 132 who wear abnormal pulse monitors 17, and an ID is added to state information to be transmitted in communication with abnormality receiving means 122, so that it is decided which of the users 132 transmitted the state information. Based on the decision results, the abnormality receiving means 122 transmits state signals separately to registered information destinations such as the doctor or family of the user 132. On the PHS 131 having received the information, the pulse abnormality of the user 132 can be remotely recognized by using a specific ringtone or vibrating function. The PHS is illustrated as an alarm receiver on the assumption that the receiver is used in a hospital and so on. The alarm receiver may be other kinds of terminals such as a mobile phone and a pager that use a public line network. As a matter of course, the alarm receiver may be constructed as an independent system using a dedicated line or radio communication such as ham radio.

### (Embodiment 4)

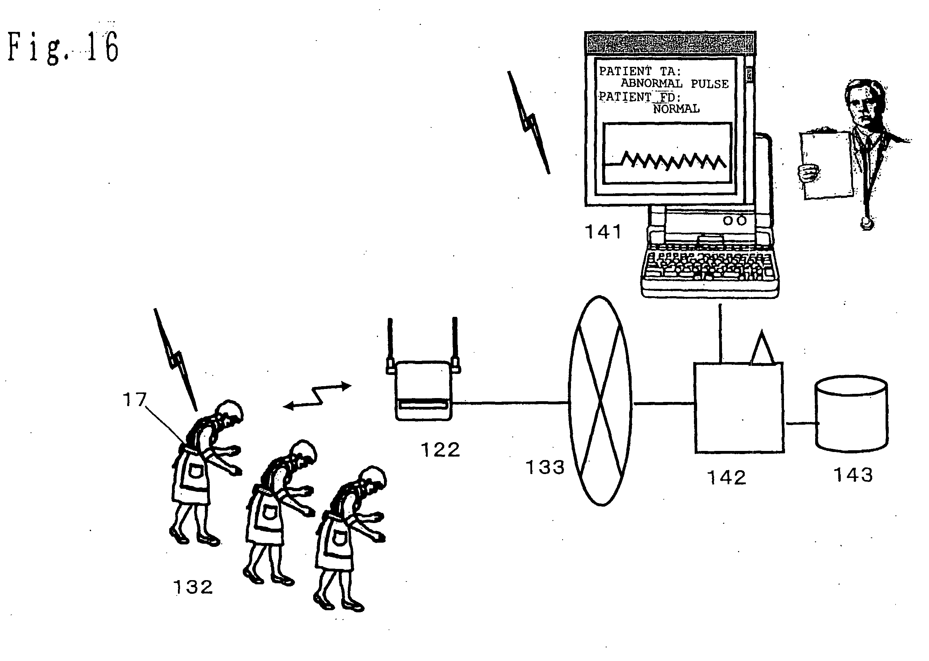

An abnormal pulse monitor of Embodiment 4 will be discussed below in accordance with the accompanying drawings. Figure 16 is a schematic structural diagram showing an abnormal pulse alarming system according to Embodiment 4 of the present invention. The configuration of Embodiment 4 will be discussed below.

In Embodiment 4, an Internet browser 141 on a PC is set as an alarm receiver of the system shown in Figure 14. Abnormality receiving means 122 accumulates, via a provider having contracted beforehand, received state information and information about which user transmitted the state information, in a server on the provider. It is possible to refer to the transmitted state information and user information on an opened web page on the Internet. In this case, the doctor or family of a user 132 make an access to the web page to refer to the state information. The state information is protected upon log-in by a password which is recognized only by registered persons. The doctor or family of the user inputs the password when referring to the state information, so that the web page is used while protecting privacy.

It is assumed in the present embodiment that abnormality information is listed on the web page. As a matter of course, the information may include posture information, an activity amount, and pulse information and may be constructed so as to confirm, e.g., a changing state on time series. Further, although the web page is used in the present embodiment, as a matter of course, a method of providing information via an exclusive push contents distribution service is also applicable.

### Industrial Applicability

With the above-described invention, it is possible to automatically judge pulse abnormality with high accuracy, which is an effective method having not been available up to this time.

## Claims

1. An abnormal pulse monitor, **characterized by** comprising:
pulse wave measuring means of measuring a pulse wave of a body,
pulse calculating means of calculating a pulse of the body based on a waveform of the pulse wave measured by the pulse wave measuring means;
acceleration detecting means of detecting an acceleration of the body,
posture deciding means which detects and decide a posture of the body based on the acceleration detected by the acceleration detecting means, and
abnormality judging means of judging abnormality based on predetermined judgment information from pulse information calculated by the pulse calculating means and posture information decided by the posture deciding means.

2. An abnormal pulse monitor **characterized by** comprising:
pulse wave measuring means of measuring a pulse wave of a body,
pulse calculating means of calculating a pulse of the body based on a waveform of the pulse wave measured by the pulse wave measuring means,
acceleration detecting means of detecting an acceleration of the body,
posture deciding means of deciding a posture of the body based on the acceleration detected by the acceleration detecting means,
activity amount judging means of judging an activity amount based on the acceleration, and
abnormality judging means of judging abnormality based on predetermined judgment information from a combination of the calculated pulse information, the detected and judged posture information, and the activity amount.

3. The abnormal pulse monitor according to any one of claims 1 and 2, **characterized by** further comprising notifying means of notifying a device user of a judgment result from the abnormality judging means.

4. The abnormal pulse monitor according to any one of inventions 1 to 3, **characterized in that** the abnormality judging means judges abnormality when the pulse calculated by the pulse calculating means is larger than a first pulse threshold value predetermined according to the posture information judged by the posture judging means.

5. The abnormal pulse monitor according to any one of inventions 1 to 4, **characterized in that** the abnormality judging means judges abnormality when the pulse calculated by the pulse calculating means is smaller than a second pulse threshold value predetermined according to the posture information obtained by the posture judging means.

6. The abnormal pulse monitor according to any one of inventions 2 and 3, **characterized in that** the abnormality judging means judges abnormality when the pulse calculated by the pulse calculating means is larger than a fifth pulse threshold value predetermined according to a combination of the posture information obtained by the posture judging means and the activity amount obtained by the activity amount judging means.

7. The abnormal pulse monitor according to any one of inventions 2 and 3, **characterized in that** the abnormality judging means judges abnormality when the pulse calculated by the pulse calculating means is smaller than a sixth pulse threshold value predetermined according to a combination of the posture information obtained by the posture judging means and the activity amount obtained by the activity amount judging means.

8. The abnormal pulse monitor according to any one of inventions 2, 3, 6, and 7, **characterized in that** the monitor further comprises information storage means of temporarily storing the judgment result, and the abnormality judging means judges the pulse to be abnormal based on a time series variation of the judgment result stored in the information storage means.

9. The abnormal pulse monitor according to any one of inventions 1, 2 to 5, 6, and 7 , **characterized in that** the monitor further comprises information storage means of temporarily storing the judgment result, and the abnormality judging means judges the pulse to be abnormal based on a frequency in a specified time of the judgment result stored in the information storage means.

10. The abnormal pulse monitor according to any one of inventions 1 to 9, **characterized by** further comprising abnormality transmitting means of transmitting abnormality judgment information when the abnormality judging means judges abnormality.

11. An abnormal pulse alarming system, **characterized by** comprising the abnormal pulse monitor of invention 10, and
abnormality receiving means of communicating with the abnormality receiving means and receiving the abnormality judgment information.

12. The abnormal pulse alarming system according to invention 11, **characterized by** further comprising the information indicating means which is connected to the abnormality receiving means and indicates the abnormality judgment information.

13. The abnormal pulse alarming system according to invention 11 or 12, **characterized by** further comprising alarming means which is connected to the abnormality receiving means and issues an alarm to a user when the abnormality receiving means receives the abnormality judgment information.

14. The abnormal pulse alarming system according to any one of inventions 11 to 13, **characterized in that** the abnormality transmitting means and the abnormality receiving means further comprise encrypting means of encrypting the abnormality judgment information.

15. The abnormal pulse alarming system according to any one of inventions 11, 12, and 14, **characterized in that** the abnormality receiving means further comprises information indicating/selecting means of indicating information on the information indicating means which is specified beforehand based on the abnormal pulse monitor having transmitted the abnormality judgment information.

16. The abnormal pulse alarming system according to any one of inventions 11, 13, and 14, **characterized in that** the abnormality receiving means further comprises alarm issuing/selecting means of issuing an alarm to the alarming means which is specified beforehand based on the abnormal pulse monitor having transmitted the abnormality judgment information.

17. The abnormal pulse alarming system according to any one of inventions 11, 12, 14, and 15, **characterized in that** the system further comprises abnormality information accumulating means which connects the abnormality receiving means and the information indicating means and accumulates the abnormality information, and a user makes an access to the abnormality information accumulating means at any given time to obtain abnormality information, so that the information indicating means indicates the abnormality information.

18. The abnormal pulse alarming system according to any one of inventions 11 to 17, **characterized in that** the abnormality judgment information includes at lease one of the pulse information, the posture information, and the activity amount information.

19. The abnormal pulse monitor according to invention 1 or 2, **characterized in that** when the posture judging means or the activity amount judging means finds a change in posture or activity amount, the abnormality judging section stops judgment for a certain time period from the change.

20. A program of causing a computer to function as the pulse calculating means of calculating a pulse of a body based on a waveform of a pulse wave measured by the pulse wave measuring means, the posture judging means of judging a posture of the body based on an acceleration detected by the acceleration detecting means, the activity amount judging means of judging an activity amount based on the acceleration, and abnormality judging means of judging abnormality based on judgment information predetermined according to a combination of the calculated pulse information, the detected and judged posture information, and the activity amount, of the abnormal pulse monitor accoring to invention 2.

21. A recording medium carrying the program of invention 20, the recording medium being processed by a computer.
